(19) Europäisches Patentamt
European Patent Office
Office européen des brevets

(11) Veröffentlichungsnummer: **0 058 250**
**A2**

# (12) EUROPÄISCHE PATENTANMELDUNG

(21) Anmeldenummer: 81110268.0

(22) Anmeldetag: 09.12.81

(51) Int. Cl.³: **C 07 D 501/36**
**A 61 K 31/545**

(30) Priorität: 17.02.81 CH 1030/81

(43) Veröffentlichungstag der Anmeldung: 25.08.82 Patentblatt 82/34

(84) Benannte Vertragsstaaten: BE CH DE FR GB IT LI NL SE

(71) Anmelder: F. HOFFMANN-LA ROCHE & CO. Aktiengesellschaft

CH-4002 Basel(CH)

(72) Erfinder: Angehrn, Peter, Dr.
Bündtenweg 27
CH-4461 Böckten(CH)

(72) Erfinder: Reiner, Roland, Dr.
Rheinfelderstrasse 8
CH-4058 Basel(CH)

(74) Vertreter: Martin, Björn et al,
Grenzacherstrasse 124 Postfach 3255
CH-4002 Basel(CH)

## (54) Cephalosporinderivate, deren Herstellung und entsprechende pharmazeutische Präparate.

(57) Cephalosporinderivate der allgemeinen Formel

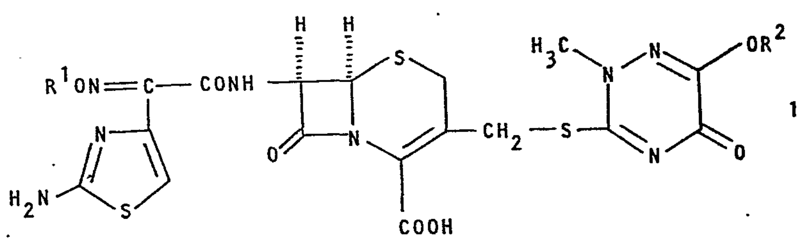

I

in der $R^1$ Carboxy-niederes Alkyl und $R^2$ Wasserstoff, ein Kation oder (mit dem Sauerstoffatom) einen leicht hydrolysierbaren Aetherrest darstellt,

sowie leicht hydrolysierbare Ester und Salze dieser Verbindungen und Hydrate der Verbindungen der Formel I und von deren Estern und Salzen, sowie deren Herstellung und entsprechende pharmazeutische Präparate.

EP 0 058 250 A2

F.Hoffmann-La Roche & Co.Aktiengesellschaft,Basel,Schweiz

RAN 4410/152

# Cephalosporinderivate, deren Herstellung und entsprechende pharmazeutische Präparate

Die vorliegende Erfindung betrifft neue Cephalosporinderivate der allgemeinen Formel

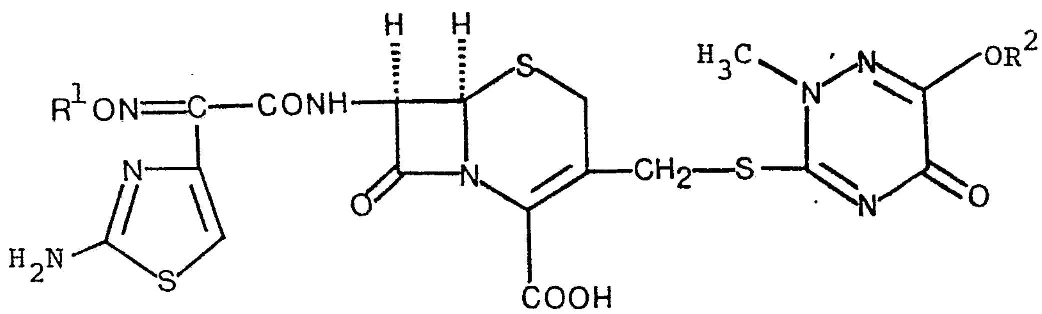

I

in der $R^1$ Carboxy - niederes Alkyl und $R^2$ Wasserstoff, ein Kation oder (mit dem Sauerstoffatom) einen leicht hydrolysierbaren Aetherrest darstellt,

sowie leicht hydrolysierbare Ester und Salze dieser Verbindungen und Hydrate der Verbindungen der Formel I und von deren Estern und Salzen.

Der Ausdruck "Alkyl" bzw. "niederes Alkyl" in den vorliegenden Zusammensetzungen stellt einen geradkettigen oder verzweigten niederen Alkylrest mit bis zu 7, vorzugsweise bis zu 4 Kohlenstoffatomen dar; z.B. Methyl, Aethyl,

Mn/17.11.81

n-Propyl, Isopropyl, n-Butyl, Isobutyl, sec-Butyl, n-Pentyl, n-Heptyl. Im Rest "Carboxy-niederes Alkyl" kann sich die Carboxygruppe an beliebiger Stelle des Alkylrestes befinden, wie z.B. in 1-Carboxyäthyl, 2-Carboxyäthyl, 1-Carboxy-1-methyl-äthyl, 2-Carboxy-1-methyl-äthyl, 1-Carboxy-1-methyl-n-propyl, Carboxymethyl. Bevorzugt ist 1-Carboxy-1-methyl-äthyl.

$R^2$ ist bevorzugt Wasserstoff oder ein Kation, kann aber auch zusammen mit dem benachbarten Sauerstoffatom eine leicht hydrolysierbare Aethergruppe darstellen, d.h. eine leicht zur Hydroxygruppe hydrolysierbare Gruppe. $R^2$ kann somit ausser Wasserstoff bzw. einem Kation z.B. folgende Gruppen darstellen: Niederes Alkanoyloxyalkyl, z.B. Acetoxymethyl, Pivaloyloxymethyl, 1-Acetoxyäthyl und 1-Pivaloyloxyäthyl; niederes Alkoxycarbonyloxyalkyl, z.B. Methoxycarbonyloxymethyl, 1-Aethoxycarbonyloxyäthyl und 1-Isopropoxycarbonyloxyäthyl; Lactonylgruppen, z.B. Phthalidyl und Thiophthalidyl; niederes Alkoxymethyl, z.B. Methoxymethyl; und niederes Alkanoylaminomethyl, z.B. Acetamidomethyl.

Als leicht hydrolysierbare Ester der Verbindungen der Formel I sind Verbindungen der Formel I zu verstehen, deren Carboxygruppe in Form einer leicht hydrolysierbaren Estergruppe vorliegt. Beispiele solcher Ester, die herkömmlicher Art sein können, sind die niederen Alkanoyloxyalkylester, z.B. der Acetoxymethyl-, Pivaloyloxymethyl-, 1-Acetoxyäthyl- und 1-Pivaloyloxyäthylester; die niederen Alkoxycarbonyloxyalkylester, z.B. der Methoxycarbonyloxymethyl-, 1-Aethoxycarbonyloxyäthyl- und 1-Isopropoxycarbonyloxyäthylester; die Lactonylester, z.B. der Phthalidyl- und Thiophthalidylester; die niederen Alkoxymethylester, z.B. der Methoxymethylester; und die niederen Alkanoylaminomethylester, z.B. der Acetamidomethylester. Auch andere Ester, z.B. die Benzyl- und Cyanmethylester, können brauchbar sein.

Beispiele von Salzen der Verbindungen der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin. Die Salze können Disalze oder auch Trisalze sein. Die dritte Salzbildung ($R^2$ = Kation) kann in Verbindungen mit dem Hydroxyrest der 2,5-Dihydro-6-hydroxy-2-methyl-5-oxo-as-triazin-3-ylgruppe auftreten, d.h. wenn $R^2$ Wasserstoff darstellt.

Wo $R^2$ Wasserstoff darstellt, steht die 2,5-Dihydro-6-hydroxy-2-methyl-5-oxo-as-triazin-3-ylgruppe in tautomerem Gleichgewicht mit der entsprechenden Diketoform, d.h. der 1,2,5,6-Tetrahydro-2-methyl-5,6-dioxo-as-triazin-3-ylgruppe.

Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Mono-arylsulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die Verbindungen der Formel I (einschliesslich deren Salze, und leicht hydrolysierbare Ester) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der synisomeren Form

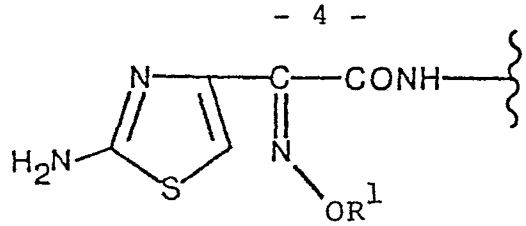

oder in der anti-isomeren Form

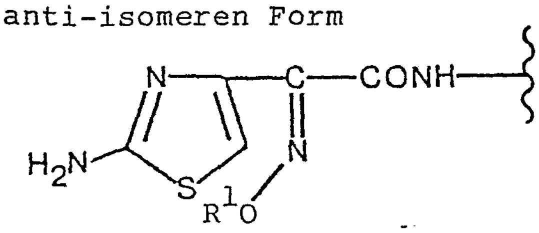

bzw. als Gemische dieser beiden Formen vorliegen. Bevorzugt ist die syn-isomere Form bzw. Gemische, in denen die syn-isomere Form überwiegt.

Bevorzugte Produkte sind

(6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure und deren Salze sowie die entsprechenden Hydrate.

Die obigen Acylderivate können erfindungsgemäss dadurch hergestellt werden, dass man

a) eine Verbindung der allgemeinen Formel

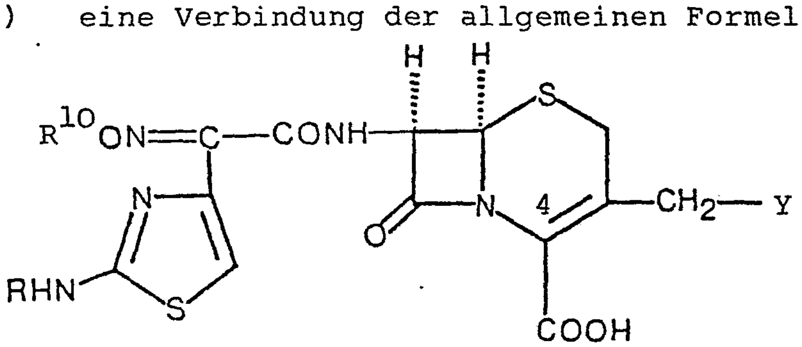

II

in der $R^{10}$ eine durch Salzbildung mit einer Base gegebenenfalls geschützte Carboxy-nieder-Alkylgruppe bedeutet, R Wasserstoff oder eine geeignete Schutzgruppe darstellt, Y eine austretende Gruppe bedeutet und die Carboxygruppe durch Salzbildung mit einer Base geschützt sein kann,

in Gegenwart von Wasser mit einem Thiol der allgemeinen Forme

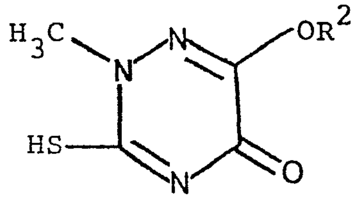

III

worin $R^2$ die oben gegebene Bedeutung hat, umsetzt, anschliessend eine allenfalls vorhandene Schutzgruppe R abspaltet und gegebenenfalls ein allenfalls erhaltenes Salz in die freie Säure überführt, oder dass man

b) zur Herstellung eines leicht hydrolysierbaren Esters einer Verbindung der Formel I eine Carbonsäure der Formel I einer entsprechenden Veresterung unterwirft, oder dass man

c) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

Die in der Ausgangsverbindung der Formel II vorhandene Carboxygruppe in Stellung 4 kann wahlweise durch Salzbildung mit einer Base geschützt sein, z.B. durch Salzbildung mit einer anorganischen Base, wie Natriumhydroxid, oder mit einer tertiären organischen Base, wie Triäthylamin. Die Carboxy-nieder-Alkylgruppe $R^{10}$ kann in gleicher Weise geschützt sein. Geeignete R-Schutzgruppen sind solche, die die Umsetzung einer Verbindung I mit dem Thiol III nicht beeinträchtigen. Beispiele von geeigneten R-Schutzgruppen sind sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Monohalogenierte Schutzgruppen, wie Chlor-, Brom- und Jodacetyl, sind für die vorliegende Thiolierungsreaktion nicht geeignet, weil sie mit dem Thiol III reagieren würden.

Als austretende Gruppe Y einer Ausgangsverbindung der Formel II kommen beispielsweise Halogene, z.B. Chlor, Brom oder Jod, Acyloxyreste, z.B. niedere Alkanoyloxyreste, wie

Acetoxy, niedere Alkyl- oder Arylsulfonyloxyreste, wie Mesyloxy oder Tosyloxy, oder der Azidorest in Frage.

Die Ausgangsverbindungen der Formel II können z.B. durch N-Acylierung der entsprechenden 7-Aminoverbindung hergestellt werden, und zwar indem man eine Verbindung der allgemeinen Formel

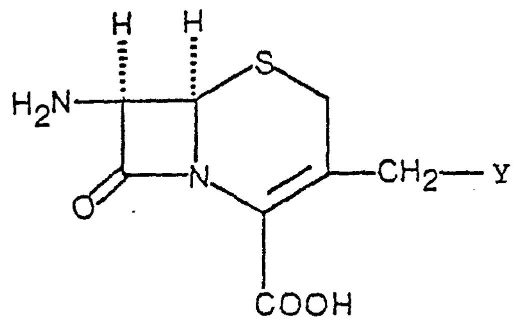

IV

in der Y die oben gegebene Bedeutung hat und die Carboxygruppe und/oder die Aminogruppe in geschützter Form vorliegen kann,

mit einer Säure der allgemeinen Formel

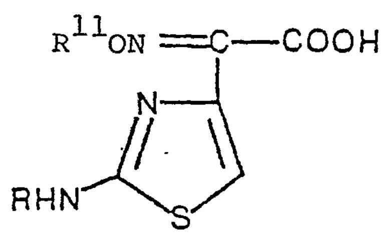

V

in der R die oben gegebene Bedeutung hat und $R^{11}$ verestertes Carboxy-niederes Alkyl darstellt,

oder mit einem reaktionsfähigen funktionellen Derivat dieser Säure umsetzt und eine allenfalls vorhandene Carboxyschutzgruppe gegebenenfalls abspaltet.

Die in der 7-Aminoverbindung der Formel IV vorhandene Carboxygruppe kann wahlweise geschützt sein, und zwar in der oben für die herzustellende Ausgangsverbindung der Formel II erläuterten Weise oder auch durch Veresterung z.B. mit einem aliphatischen oder araliphatischen Esterrest, z.B. t-Butyl, oder mit einem Silylrest, wie Trimethylsilyl. Die Aminogruppe der Verbindung der Formel IV kann z.B. durch eine Silylschutzgruppe, wie Trimethylsilyl,

geschützt sein.

Als reaktionsfähige funktionelle Derivate von Säuren der Formel V kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Der Carboxyrest der Carboxy-niederalkylgruppe $R^{11}$ der Säure der Formel V bzw. von deren reaktionsfähigen funktionellen Derivaten, liegt in veresterter Form vor, z.B. als aliphatischer oder araliphatischer Ester, z.B. als t-Butylester, oder als Silylester, z.B. als Trimethylsilylester.

Die Umsetzung der 7-Aminoverbindung der Formel IV mit der Säure der Formel V oder einem reaktionsfähigen funktionellen Derivat davon kann in an sich bekannter Weise durchgeführt werden. So kann man z.B. eine freie Säure der Formel V mit einem der erwähnten Ester entsprechend Formel IV mittels eines Carbodiimids, wie Dicyclohexylcarbodiimid, in einem inerten Lösungsmittel, wie Aethylacetat, Acetonitril, Dioxan, Chloroform, Methylenchlorid, Benzol oder Dimethylformamid, kondensieren und anschliessend die Estergruppe abspalten. Anstelle von Carbodiimiden lassen sich als Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel IV, z.B. ein Trialkylammoniumsalz, wie das Triäthylammoniumsalz, mit einem reaktionsfähigen funktionellen Derivat einer Säure der Formel V wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel V mit dem Amin der Formel IV umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, gegebenenfalls im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel IV wird in wasserfreiem Medium gearbeitet.

Die Umsetzung der 7-Aminoverbindung der Formel IV mit der Säure der Formel V oder einem reaktionsfähigen funktionellen Derivat davon kann zweckmässig bei Temperaturen zwischen etwa -40°C und Zimmertemperatur, beispielsweise bei etwa 0-10°C, erfolgen.

In der Ausgangsverbindung II vorhandene Schutzgruppen (R, $R^{10}$ oder an der 4-Carboxygruppe) werden vor der Durchführung der erfindungsgemässen Thiolierung mit den Thiolen III vorzugsweise abgespalten. Es ist auch möglich, die Schutzgruppen nach der Thiolierung abzuspalten, wobei jedoch in jedem Fall vorhandene Estergruppen an 4-Carboxy entfernt werden sollen, damit die Thiolierung in guter Ausbeute durchgeführt werden kann. Die Abspaltung der verschiedenen Schutzgruppen kann sowohl durch saure als auch durch alkalische Hydrolyse erfolgen. Saure Hydrolyse, vorzugsweise mit Hilfe einer niederen, gegebenenfalls halogenierten Alkancarbonsäure, z.B. Ameisensäure, Trifluoressigsäure, entfernt die sauer-hydrolytisch abspaltbaren Schutzgruppen R, z.B. t-Butoxycarbonyl und Trityl. Basische Hydrolyse, vorzugsweise mit Hilfe verdünnter wässriger

Alkalilauge, entfernt die basisch-hydrolytisch abspaltbaren Schutzgruppen R, z.B. Trifluoracetyl. Die Esterschutzgruppen an $R^{11}$ bzw. an 4-Carboxy können sowohl sauer als auch basisch abgespalten werden. Silylschutzgruppen werden leicht durch Behandeln mit Wasser abgespalten. Die Temperatur für die Abspaltung der verschiedenen Schutzgruppen ist in der Regel die Raumtemperatur, obwohl auch leicht erhöhte bzw. leicht erniedrigte Temperaturen angewendet werden können, z.B. etwa 0°C bis +40°C.

Anschliessend können die so freigesetzten Carboxygruppen gegebenenfalls durch Behandlung mit einer anorganischen oder organischen Base, z.B. mit Natriumhydroxid, Natrium-2-äthylcaproat oder Triäthylamin, wieder geschützt werden.

Das als Ausgangsverbindung eingesetzte Thiol der Formel III steht in tautomerem Gleichgewicht mit dem entsprechenden Thion:

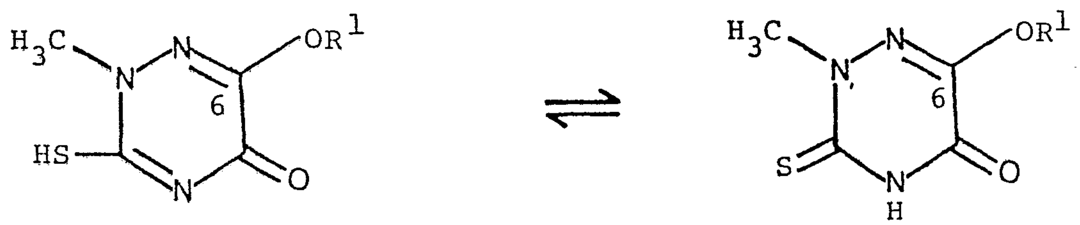

III III a

Das Thiol III, worin $R^1$ Wasserstoff darstellt, ist bekannt. Zur Herstellung von in 6-Stellung verätherten Thiolen III wird im allgemeinen die $R^1$-Gruppe eingeführt, indem man ein S-geschütztes Thiol (z.B. durch Benzhydryl) mit dem die $R^1$-Gruppe enthaltenden Halogenid, vorzugsweise dem Jodid, in einem inerten organischen Lösungsmittel in Gegenwart eines säurebindenden Mittels, z.B. Kaliumcarbonat, umsetzt, vorzugsweise bei etwa 10°-50°C, und die Schutzgruppe abspaltet (Benzhydryl kann mit Anisol und Trifluoressigsäure bei Raumtemperatur abgespalten werden).

Die erfindungsgemässe Thiolierung der Ausgangsverbindung der Formel II mit dem Thiol der Formel III kann in an sich bekannter Weise, z.B. bei einer Temperatur zwischen etwa 40 und 80°C, zweckmässig bei etwa 60°C, in Wasser oder in einer Pufferlösung mit einem pH von etwa 6 bis 7, vorzugsweise 6,5, durchgeführt werden. Eine allenfalls vorhandene Schutzgruppe R wird anschliessend abgespalten, und zwar in der oben angegebenen Weise. Ein allenfalls erhaltenes Salz (Mono-, Di- oder Trisalz, d.h. Salzbildung ist möglich an $R^1$, $R^2$ und an der 4-Carboxygruppe) kann durch Neutralisation mit einer geeigneten Säure ganz oder teilweise in die freie Dicarbonsäure bzw. das freie Enol ($R^2$ = Wasserstoff) übergeführt werden. Als Säure kommt z.B. Salzsäure, Schwefelsäure, Phosphorsäure oder Citronensäure in Betracht.

Zur Herstellung der leicht hydrolysierbaren Ester der Dicarbonsäure der Formel I gemäss Variante b) wird die Dicarbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin, beschleunigt werden. Je nach der verwendeten Menge Veresterungsmittel können Mono- oder Diester erhalten werden. Falls die Gruppe $OR^2$ die Hydroxygruppe darstellt, wird diese unter diesen Bedingungen unter Bildung eines entsprechenden, leicht hydrolysierbaren Aethers veräthert. Vorzugsweise verwendet man dabei einen Ueberschuss an dem entsprechenden Halogenid. Die Veresterungs-/Verätherungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0-40°C.

Die Herstellung der Salze und Hydrate der Verbindungen der Formel I bzw. der Hydrate dieser Salze kann in an sich bekannter Weise erfolgen, z.B. durch Umsetzung der Dicarbonsäure der Formel I mit der gewünschten Base, zweckmässig in einem Lösungsmittel, wie Wasser oder in einem organischen Lösungsmittel, wie Aethanol, Methanol, Aceton und anderen mehr. Es können je nach der verwendeten Menge Base Mono- oder Di-salze erhalten werden. Bei Verwendung einer Verbindung I mit $OR^2$=OH kann Salzbildung auch an dieser Hydroxygruppe erfolgen, wobei ein Trisalz entsteht. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Bildung von Hydraten erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester oder Salz davon) einer feuchten Atmosphäre, z.B. bei etwa +10°C bis +40°C, ausgesetzt werden.

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisieren oder durch chromatographische Methoden unter Verwendung eines geeigneten Lösungsmittels bzw. Lösungsmittelgemisches.

Die Verbindungen der Formel I sowie die entsprechenden leicht hydrolysierbaren Ester und Salze bzw. die Hydrate dieser Produkte sind antibiotisch, insbesondere bakterizid wirksam. Sie besitzen ein breites Wirkungsspektrum gegen verschiedene Gram-positive und insbesondere gegen Gram-negative Mikroorganismen, einschliesslich β-Lactamase-bildende Gram-negative Bakterien, wie z.B. Pseudomonas aeruginosa, Haemophilus influenzae, Escherichia coli, Serratia

marcescens, Proteus- und Klebsiella-Spezies.

Die erfindungsgemässen Produkte können dementsprechend zur Behandlung und Prophylaxe von Infektionskrankheiten verwendet werden. Für den Erwachsenen kommt eine Tagesdosis von etwa 0,1 g bis etwa 2 g in Betracht. Die parenterale Verabreichung der erfindungsgemässen Verbindungen ist besonders bevorzugt.

Zum Nachweis der antimikrobiellen Wirksamkeit der erfindungsgemässen Produkte wurde ein repräsentativer Vertreter, nämlich das Trinatriumsalz der (6R,7R)-7-[(Z)-2-(2-Amino-4-thiazolyl)-2-[(1-carboxy-1-methyläthoxy)imino]-acetamido]-3-[[(2,5-dihydro-6-hydroxy-2-methyl-5-oxo-as-triazin-3-yl)thio]methyl]-8-oxo-5-thia-1-azabicyclo[4.2.0]-oct-2-en-2-carbonsäure auf seine in-vitro-Aktivität gegen 15 Stämme des humanpathogenen Krankheitserregers Pseudomonas aeruginosa getestet.

Ergebnis (als MIC=Mindesthemmkonzentration in µg/ml):

| Bezeichnung des Stammes von P.aeruginosa | MIC µg/ml |
|---|---|
| 1 | 6,3 |
| 2 | 1,6 |
| 3 | 1,6 |
| 4 | 1,6 |
| 5 | 1,6 |
| 6 | 1,6 |
| 7 | 1,6 |
| 8 | 25 |
| 9 | 3,1 |
| 10 | 1,6 |
| 11 | 0,8 |
| 12 | 1,6 |
| 13 | 1,6 |
| 14 | 1,6 |
| 15 | 1,6 |

Toxizität des Trinatriumsalzes der (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure:

| | | |
|---|---|---|
| $LD_{50}$, mg/kg | i.v. | >1000 |
| | s.c. | >4000 |
| | p.o. | >4000. |

Die erfindungsgemässen Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutisch wertvolle Stoffe enthalten. Die Verbindungen der Formel I, worin $R^2$ Wasserstoff darstellt, und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien, wie Wasser oder isotonischer Kochsalzlösung, zubereitet. Die entsprechenden leicht hydrolysierbaren Ester und ihre Salze bzw. Hydrate kommen auch für die enterale Verabreichung in Betracht.

Beispiel 1

Herstellung des Trinatriumsalzes der (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure.

17,13 g Dinatriumsalz der (6R,7R)-3-(Acetoxymethyl)-7-[(Z)-2-(2-amino-4-thiazolyl)-2-[(1-carboxy-1-methyläthoxy)imino]acetamido]-8-oxo-5-thia-1-azabicyclo[4.2.0]oct-2-en-2-carbonsäure werden in 200 ml Wasser gelöst und mit 5,25 g Tetrahydro-2-methyl-3-thioxo-as-triazin-5,6-dion versetzt. Das Gemisch wird 10 Stunden bei 55-60°C unter Stickstoff-Begasung gerührt, wobei das pH mit 1N Natronlauge bei 6,5 mittels Autotitrator gehalten wird. Die rötliche Lösung wird auf 25°C gekühlt und mit 150 ml Wasser verdünnt. Das pH wird unter Rühren mit ca. 62 ml 1N Salzsäure auf 3,8 gestellt. Das ausgefallene Material wird abgenutscht, mit 50 ml Wasser gewaschen, gut trocken gesaugt und im Vakuum bei 25°C getrocknet. Man erhält eine bräunliche Fraktion I, welche verworfen wird. Die vereinigten Mutterlauge und Waschwasser (gelb-orange) werden mit ca. 38 ml 1N Salzsäure unter Rühren auf pH 3 gestellt. Das ausgefallene Material wird abgenutscht, mit 100 ml Wasser gewaschen, gut trocken gesaugt und im Vakuum bei 25°C getrocknet. Man erhält eine beige Fraktion II, welche zwecks Bildung des Trinatriumsalzes in 1500 ml Methanol mit 30 ml einer 2N Lösung von 2-Aethylcapronsäure-Natriumsalz in Aethylacetat versetzt wird. Das Gemisch wird 15 Minuten gerührt, wobei eine Lösung entsteht. Nach Zugabe von Aethanol bis zur leichten Trübung wird im Vakuum bei 40°C stark eingeengt, wobei Substanz ausfällt. Diese wird abgenutscht, nacheinander mit Aethanol und tiefsiedendem Petroläther gewaschen und über Nacht im Hochvakuum bei 45°C getrocknet. Danach wird die Substanz noch 1 Stunde an der Luft äquilibriert. Man erhält beige, amorphe Titelsubstanz mit $[\alpha]_D^{20}$ = -81° (c=1 in Wasser). Das Kernresonanzspektrum entspricht der angegebenen

Struktur.

Das als Ausgangsmaterial verwendete Dinatriumsalz der (6R,7R)-3-(Acetoxymethyl)-7-[(Z)-2-(2-amino-4-thiazolyl)-2-[(1-carboxy-1-methyläthoxy)imino]acetamido]-8-oxo-5-thia-1-azabicyclo[4.2.0]oct-2-en-2-carbonsäure kann wie folgt hergestellt werden:

5,72 g 2-(Tritylamino)-4-thiazolbrenztraubensäure-(Z)-O-[1-(tert-butoxycarbonyl)-1-methyläthyl]oxim und 3,28 g 7-Aminocephalosporansäure-tert.-butylester werden zu einem Gemisch von 1 ml Pyridin und 100 ml Dichlormethan bei 25°C gegeben. Zur entstandenen Lösung werden 2,27 g Dicyclohexylcarbodiimid hinzugefügt. Das Reaktionsgemisch wird 1 Stunde bei 25°C gerührt, wobei Dicyclohexylharnstoff ausfällt. Dieser wird abfiltriert und das gelbe Filtrat im Vakuum bei 40°C eingedampft. Der Rückstand wird in 100 ml Aethylacetat aufgenommen, nacheinander mit 50 ml 1N Salzsäure, 50 ml verdünnter Natriumchlorid-Lösung und 50 ml gesättigter Natriumhydrogencarbonat-Lösung gewaschen, über Natriumsulfat getrocknet und im Vakuum bei 40°C eingedampft. Man erhält einen rohen, gelblichen Schaum als Eindampfrückstand, welcher zwecks Reinigung an einer Kieselgelsäule mit Aether als Elutionsmittel gereinigt wird. Die das gewünschte Produkt enthaltenden Fraktionen werden gesammelt und im Vakuum eingedampft. Der verbleibende Schaum wird in 200 ml Isopropyläther aufgekocht, wobei die Substanz zunächst in Lösung geht und kurz darauf auskristallisiert. Das Kristallisationsgemisch lässt man auf 25°C abkühlen. Das Kristallisat wird abgenutscht, mit Isopropyläther und tiefsiedendem Petroläther nacheinander gewaschen und über Nacht im Hochvakuum bei 40°C getrocknet. Man erhält (6R,7R)-3-(Acetoxymethyl)-7-[(Z)-2-[[1-(tert-butoxycarbonyl)-1-methyläthoxy]imino]-2-[2-(tritylamino)-4-thiazolyl]acetamido]-8-oxo-5-thia-1-azabicyclo[4.2.0]oct-2-en-2-carbonsäure-tert-butylester als weisse Kristalle mit $[\alpha]_D^{25}$ = +5,6° (c=1 in Dimethylsulfoxid). Das Kernresonanzspektrum und die Mikroanalyse entsprechen der angegebenen Struktur.

62 g (6R,7R)-3-(Acetoxymethyl)-7-[(Z)-2-[[1-(tert-butoxycarbonyl)-1-methyläthoxy]imino]-2-[2-(tritylamino)-4-thiazolyl]acetamido]-8-oxo-5-thia-1-azabicyclo[4.2.0]oct-2-en-2-carbonsäure-tert-butylester werden in 500 ml Trifluoressigsäure gelöst. Die Lösung wird nach anderthalbstündigem Rühren bei 25°C im Vakuum bei 40°C eingedampft. Der Rückstand wird 2 Stunden im Hochvakuum bei 40°C unter Verwendung eines Trockeneiskühlers eingedampft. Der verbleibende, gelbbraune Schaum wird in 500 ml reiner Ameisensäure gelöst. Dieser Lösung werden 150 ml Wasser zugegeben; das erhaltene Gemisch wird 1 1/4 Stunden bei 25°C gerührt. Dem Gemisch werden weitere 500 ml Wasser hinzugefügt. Das ausgefallene Triphenylcarbinol wird durch Filtration abgetrennt. Das gelblich-braune Filtrat wird im Vakuum bei 40°C eingedampft. Der verbleibende ölige Rückstand wird mit 200 ml Wasser versetzt. Wenig braunes, ungelöstes Material wird abfiltriert und verworfen. Das gelblich-braune Filtrat wird über Nacht lyophilisiert. Man erhält ein gelbliches Lyophilisat, welches zwecks Bildung des Dinatriumsalzes in 600 ml Methanol gelöst und mit 100 ml einer 2N Lösung von 2-Aethylcapronsäure-Natriumsalz in Aethylacetat versetzt wird. Nach Zugabe von 600 ml Aethanol bis zur leichten Trübung wird das Gemisch im Vakuum bei 40°C stark eingeengt, wobei Substanz ausfällt. Diese wird abgenutscht, nacheinander mit Aethanol und tiefsiedendem Petroläther gewaschen und über Nacht im Vakuum bei 40°C getrocknet. Man erhält eine beige Fraktion I. Die Mutterlauge wird mit dem Waschäthanol vereinigt und das Gemisch mit 500 ml Aethanol versetzt und im Vakuum bei 40°C wieder eingeengt. Das amorph ausgefallene Material wird abgenutscht, nacheinander mit Aethanol und tiefsiedendem Petroläther gewaschen und über Nacht im Vakuum bei 40°C getrocknet. Man erhält eine hellbeige Fraktion II. Zwecks Reinigung werden die vereinigten Fraktionen I und II in 500 ml Methanol gelöst, mit 25 ml einer 2N Lösung von 2-Aethylcapronsäure-Natriumsalz in Aethylacetat versetzt und das Gemisch mit 750 ml Aethanol verdünnt. Das dabei amorph ausgefallene, bräunliche Material wird abfiltriert und verworfen. Das dunkelgelbe Filtrat wird im Vakuum bei 40°C stark eingeengt, mit 500 ml Aethanol verdünnt und wieder im Vakuum bei 40°C eingeengt. Das dabei auskristallisierte Material wird abgenutscht, nacheinander mit Aethanol und tiefsiedendem Petroläther gewaschen und über Nacht im Hochvakuum bei 40-45°C getrocknet. Man erhält das Dinatriumsalz der (6R,7R)-3-(Acetoxymethyl)-7-[(Z)-2-(2-amino-4-thiazolyl)-2-[(1-carboxy-1-methyläthoxy)imino]acetamido]-8-oxo-5-thia-1-azabicyclo[4.2.0]oct-2-en-2-carbonsäure als gelbliche Kristalle mit $[\alpha]_D^{25}$ = +40,7° (c=1 in Wasser). Das Kernresonanzspektrum entspricht der angegebenen Struktur.

## Beispiel 2

Herstellung von Trockenampullen für die intramuskuläre Verabreichung:

Es wird in üblicher Weise ein Lyophilisat von 1 g des Trinatriumsalzes der (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure hergestellt und in eine Ampulle abgefüllt. Vor der Verabreichung wird das Lyophilisat mit 2,5 ml einer 2%igen wässrigen Lidocainhydrochlorid-Lösung versetzt.

## Patentansprüche

1. Cephalosporinderivate der allgemeinen Formel

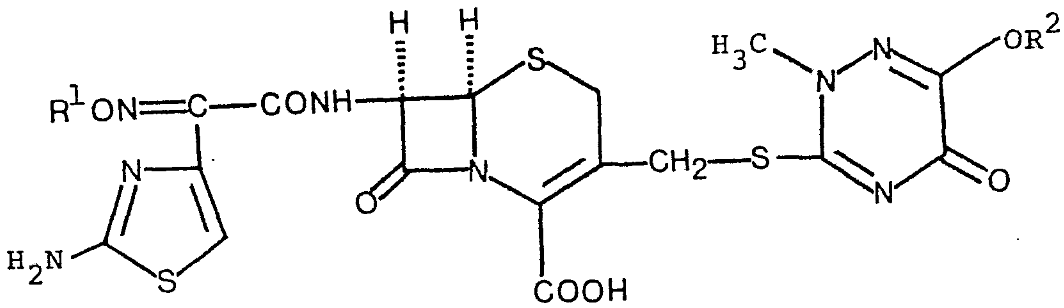

I

in der $R^1$ Carboxy-niederes Alkyl und $R^2$ Wasserstoff, ein Kation oder (mit dem Sauerstoffatom) einen leicht hydrolysierbaren Aetherrest darstellt,

sowie leicht hydrolysierbare Ester und Salze dieser Verbindungen und Hydrate der Verbindungen der Formel I und von deren Estern und Salzen.

2. Cephalosporinderivate nach Anspruch 1, in der syn-isomeren Form bzw. Gemische, in denen die syn-isomere Form überwiegt.

3. Cephalosporinderivate nach Anspruch 1 oder 2, dadurch gekennzeichnet, dass $R^1$ 1-Carboxy-1-methyläthyl darstellt.

4. Cephalosporinderivate nach einem der Ansprüche 1-3, dadurch gekennzeichnet, dass $R^2$ Wasserstoff darstellt.

5. Das Trinatriumsalz eines Cephalosporinderivats gemäss Anspruch 4.

6. (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure sowie Salze dieser Verbindung und Hydrate dieser Verbindung bzw. Salze.

7. Verbindungen gemäss einem der Ansprüche 1-6 als pharmazeutische Wirkstoffe.

8. (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure sowie Salze dieser Verbindung und Hydrate dieser Verbindung bzw. Salze als pharmazeutische Wirkstoffe.

9. Verbindungen gemäss einem der Ansprüche 1-6 als pharmazeutische Wirkstoffe zur Behandlung und Prophylaxe von Infektionskrankheiten.

10. (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure sowie Salze dieser Verbindung und Hydrate dieser Verbindung bzw. Salze als pharmazeutische Wirkstoffe zur Behandlung und Prophylaxe von Infektionskrankheiten.

11. Pharmazeutische Präparate, gekennzeichnet durch einen Gehalt an einer Verbindung gemäss einem der Ansprüche 1-6.

12. Präparat gemäss Anspruch 11, dadurch gekennzeichnet, dass es (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure, ein Salz dieser Verbindung oder ein Hydrat dieser Verbindung bzw. dieses Salzes enthält.

13. Pharmazeutische Präparate zur Behandlung und Prophylaxe von Infektionskrankheiten, gekennzeichnet durch einen Gehalt an einer Verbindung gemäss einem der Ansprüche 1-6.

14. Präparat gemäss Anspruch 13, dadurch gekennzeichnet, dass es (6R,7R)-7-[(Z)-2-(2-Amino-4-thiazolyl)-2-[(1-carboxy-1-methyläthoxy)imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure, ein Salz dieser Verbindung oder ein Hydrat dieser Verbindung bzw. dieses Salzes enthält.

15. Verfahren zur Herstellung der Verbindungen gemäss einem der Ansprüche 1-6, dadurch gekennzeichnet, dass man

a) eine Verbindung der allgemeinen Formel

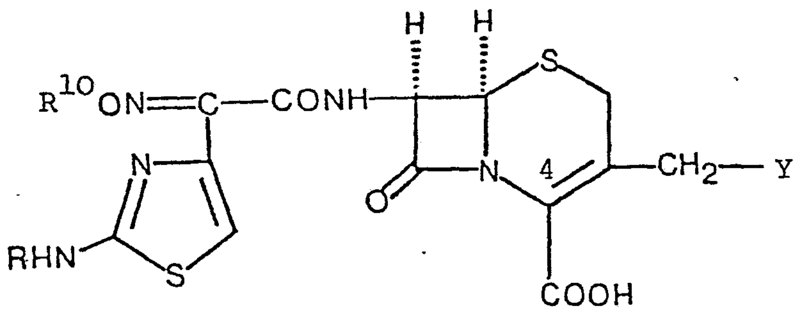

II

in der $R^{10}$ eine durch Salzbildung mit einer Base gegebenenfalls geschützte Carboxy-nieder-Alkylgruppe bedeutet, R Wasserstoff oder eine geeignete Schutzgruppe darstellt, Y eine austretende Gruppe bedeutet und die Carboxygruppe durch Salzbildung mit einer Base geschützt sein kann,

in Gegenwart von Wasser mit einem Thiol der allgemeinen Formel

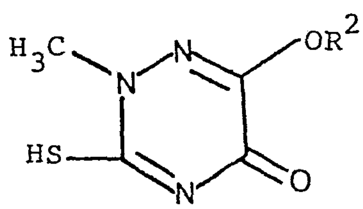

III

worin $R^2$ die in Anspruch 1 gegebene Bedeutung hat, umsetzt, anschliessend eine allenfalls vorhandene Schutzgruppe R abspaltet und gegebenenfalls ein allenfalls erhaltenes Salz in die freie Säure überführt, oder dass man

b) zur Herstellung eines leicht hydrolysierbaren Esters einer Verbindung der Formel I eine Carbonsäure der Formel I einer entsprechenden Veresterung unterwirft, oder dass man

c) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

***